# EUROPÄISCHE PATENTSCHRIFT

(11) **EP 1 358 862 B1**
(45) Veröffentlichungstag und Bekanntmachung des Hinweises auf die Patenterteilung: **17.11.2004**
(21) Anmeldenummer: 03009209.2
(22) Anmeldetag: 22.07.2000
(51) Int. Cl.: A61F 5/01

(54) **Orthese**
Orthesis
Orthese

(30) Priorität: 17.08.1999 DE 29914375 U
(43) Veröffentlichungstag der Anmeldung: 05.11.2003
(62) Teilanmeldung aus: 00954354.7
(73) Patentinhaber: Medi Bayreuth Weihermüller & Voigtmann GmbH & Co. KG, 95448 Bayreuth (DE)
(72) Erfinder: Weihermüller, Michael, Dr., 95448 Bayreuth (DE)
(74) Vertreter: Schuhmann, Albrecht

(56) Entgegenhaltungen:
- EP-A- 0 693 276
- EP-A- 0 841 044
- US-A- 5 031 606
- US-A- 5 036 837

## Beschreibung

Die vorliegende Erfindung betrifft ein Orthesengelenk mit den Merkmalen des Oberbegriffs des Anspruchs 1.

Ein einstellbares Orthesengelenk ist beispielsweise aus der EP-A-0 841 044 bekannt. Hier wird ein bestimmter voreinstellbarer Schwenkbereich der Orthese von einer Federkraft beaufschlagt, wobei die Feder für eine beaufschlagungsfreie Extensions- oder Flexionsbewegung außer Kraft gesetzt werden kann. Ein Orthesengelenk für Orthesen für zwei gegeneinander beug- und streckbare Körperteile, wie Knieorthesen oder Armorthesen, wobei das Orthesengelenk zwei Schienen drehbar miteinander verbindet, die jeweils mit oberen und unteren Orthesenteilen verbunden sind, die an dem jeweiligen Körperteil befestigbar sind und wobei an der Orthese jeweils lateral und/oder medial ein Schienenpaar mit Orthesengelenk angeordnet ist, wobei die Schienen endseitig über einen oder mehrere gemeinsame Zapfen oder durch endseitige Verzahnungen im Orthesengelenk miteinander in Eingriff stehen, und wobei für Streckung und/oder Beugung einstellbare Endanschläge vorhanden sind ist aus der EP-A-693 276 bekannt. Die EP-A-633 007 zeigt einen ähnlichen Aufbau, jedoch mit einem Klinkensystem.

Aufgabe der vorliegenden Erfindung ist es, ein Orthesengelenk zu schaffen, dessen Extensions- oder Flexionsbereich einstellbar ist, wobei die Bewegung in einem bestimmten Bereich beaufschlagungsfrei erfolgt, jedoch vor Erreichen des jeweiligen Endpunkts auf eine entgegen wirkende, bremsende Kraft stößt. Damit soll eine schlagartige Belastung der Bänder, insbesondere von Kreuzbändern, vermieden werden, indem der Patient vor Errreichen des Endpunkts auf spürbaren Widerstand stößt. Zugleich hat dies den Effekt, daß die Rückkehr in eine neutrale Position angeregt wird.

Diese Aufgabe wird mit den Merkmalen des kennzeichnenden Teils des Anspruchs 1 gelöst. Fortbildungen und vorteilhafte Ausführungen der Erfindung sind in den weiteren Ansprüchen umfaßt.

Erfindungsgemäß ist ein Orthesengelenk für Orthesen für zwei gegeneinander beug- und streckbare Körperteile, wie Knieorthesen oder Armorthesen, wobei das Orthesengelenk zwei Schienen drehbar miteinander verbindet, die jeweils mit oberen und unteren Orthesenteilen verbunden sind, die an dem jeweiligen Körperteil befestigbar sind und wobei an der Orthese jeweils lateral und/oder medial ein Schienenpaar mit Orthesengelenk angeordnet ist, wobei die Schienen endseitig über einen oder mehrere gemeinsame Zapfen oder durch endseitige Verzahnungen im Orthesengelenk miteinander in Eingriff stehen, und wobei für Streckung und/oder Beugung einstellbare Endanschläge vorhanden sind, dadurch gekennzeichnet daß die Endanschläge über einen oder mehrere Winkelbereiche δ einstellbar sind und daß die Beugung, bzw. Streckung in dem Orthesengelenk in einem Winkelbereich von δ + α oder δ - α eine konstante oder dynamische Bremsung erfährt, wobei α einen Winkel von zwischen 3° und 25° darstellt.

Die Bremsung erfolgt pneumatisch oder hydraulisch, wobei ein Mitnehmer ab Erreichen des bestimmten Winkelbereichs ein Pneumatik- oder Hydraulikglied, das an der Orthese gelagert ist, auslöst. Das Hydraulikglied kann auf niedrig- oder hochviskoser Basis wirken.

Die Rückbewegung in die Ausgangsposition, d.h. im wesentlichen die Geradestellung des Gelenks, erfolgt ohne Beaufschlagung durch eine Bremskraft, die nur in Extensions- oder Flexionsrichtung wirkt.

Nach einer bevorzugten Ausführung der Erfindung drehen die Endstücke der Schienen jeweils auf Zapfen einer diese verbindenden Grundplatte.

Im folgenden wird die Erfindung anhand von Zeichnungen beispielhaft näher beschrieben. Es zeigen:
Fig. 1 schematisch die Anordnung einer Orthese im Kniebereich;
Fig. 2 einen Längsschnitt durch das Orthesengelenk;
Fig. 3 eine Draufsicht auf die Mitnahme- und Bremsmechanik des Orthesengelenks gemäß dem Parallelpatent EP-B-1 121 077.
Fig. 4 eine Draufsicht auf das Orthesengelenk nach Fig. 3 unter Weglassung der oberen Abdeckplatte.

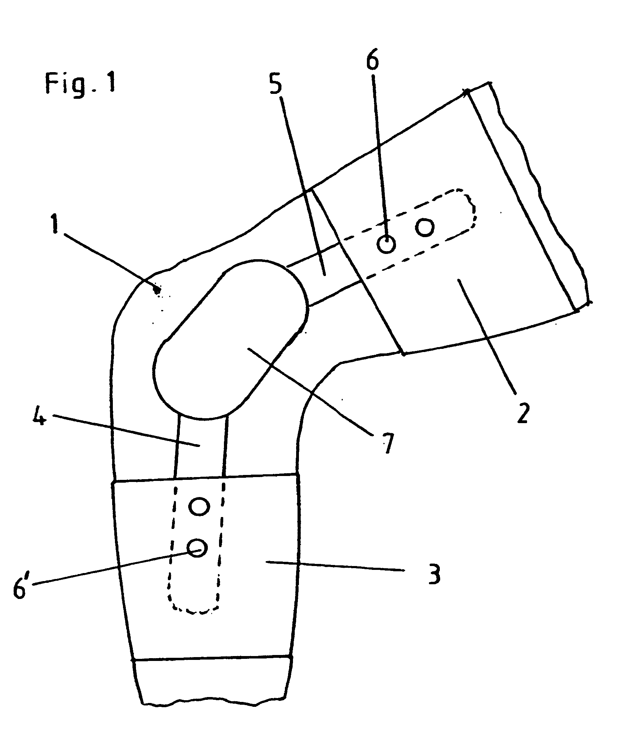

Fig. 1 zeigt ein Knie 1 mit einer angelegten Knieorthese. Die Knieorthese besteht aus einem Halteteil 2 für den Oberschenkel und einem Halteteil 3 für den Unterschenkel. In den Halteteilen sind lateral und medial jeweils Metallschienen 4, 5 mittels Nieten 6 befestigt, wobei sich die Schiene 5 des Halteteils 2 für den Oberschenkel nach unten und die Schiene 4 des Halteteils 3 für den Unterschenkel nach oben erstrecken. Beide Schienen vereinigen sich in dem Orthesengelenk 7, in dem sie über einen bestimmten Winkelbereich drehbar gelagert sind und über eine Verzahnung miteinander in Eingriff stehen.

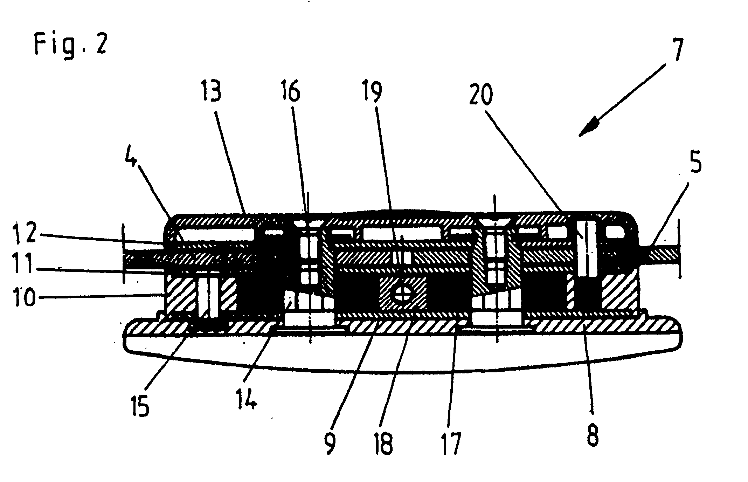

Fig. 2 zeigt den Aufbau des Orthesengelenks 7 im Längsschnitt. Es besteht aus einer Grundplatte 8, in der zwei nach oben gerichtete Sechskantzapfen 14 drehbar gelagert sind. Auf der Grundplatte liegt eine erste Zwischenplatte 9, an die sich ein flaches Gehäuse 10 anschließt. Das Gehäuse 10 beherbergt die Mitnahme- und Bremsmechanik, die weiter unten näher beschrieben wird. Das Gehäuse wird von einer zweiten Zwischenplatte 12 abgedeckt, durch die die Sechskantzapfen 14 drehbar hindurch geführt sind. Auf der zweiten Zwischenplatte 11 und unter einer dritten Zwischenplatte 12 legen die Endstücke der Schienen 4, 5, die über Verzahnungen miteinander in Eingriff stehen. Die Endstücke der Schienen 4, 5 sind drehfest mit den Sechskantzapfen 14 verbunden, die über den Schienen Schultern aufweisen, mit denen sie unter der dritten Zwischenplatte 12 anliegen, jedoch mit einem verjüngten zylindrischen Ende drehbar durch diese hindurch geführt sind. Auf der dritten Zwischenplatte 12 ist eine Abdeckplatte 13 angeordnet, die durch Schrauben 16 mit den Sechskantzapfen 14 verbunden ist, wobei sich die Schrauben 16 in der Abdeckplatte 13 drehen können. Die Grundplatte 8 ist über Schrauben oder Stifte 15 mit den unteren beiden Zwischenplatte 9, 11 und dem dazwischen liegenden Gehäuse 10 verbunden, so daß das Orthesengelenk 7 eine von oben und unten gesicherte Einheit darstellt.

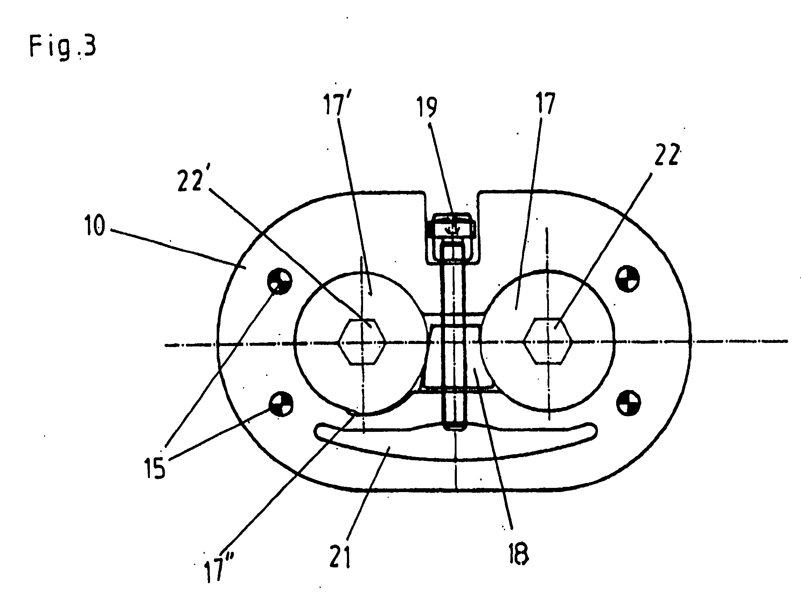

Fig. 3 zeigt eine Draufsicht auf das Gehäuse 10 mit seinen Mechanikteilen, wobei alle anderen Elemente des Orthesengelenks weggelassen sind. Zu erkennen sind vier untere Befestigungsstifte 15, die seitlich an den Enden angeordnet sind. Es sind zwei Ausnehmungen vorhanden, in denen in einem Abstand zueinander in der Längsachse des Gelenks zwei Scheiben 17, 17' angeordnet sind. Die Scheiben weisen zentrale Sechskantbohrungen 22, 22' auf, mit denen sie drehfest auf die (hier nicht gezeigten) Sechskantzapfen gesteckt sind. Solche Sechskantbohrungen weisen auch die (hier nicht gezeigten) Endstücke der Schienen auf, so daß sich, wenn sich die Schiene zueinander verdrehen, die Scheiben 17, 17' zwangsläufig mitdrehen. Die rechte Scheibe 17 ist kreisrund ausgeführt, während die linke Scheibe einen exzentrischen Abschnitt aufweist. Zwischen den Scheiben ist ein Keil 18 angeordnet, der mit einer Gewindebohrung verdrehsicher zwischen den (hier nicht gezeigten) Zwischenplatten auf einem Gewindebolzen läuft. Der Gewindebolzen 19 ist mit seinem inneren Ende an einem elastischen Element 21 gelagert, das ihn mit Federkraft beaufschlagt und nach außen zu drücken trachtet. Das elastiche Element 21 wird bei vorliegender Erfindung von einem Pneumatik- oder Hydraulikglied gebildet, das von einem Mitnehmer ab Erreichen eines bestimmten Winkelbereiches ausgelöst wird. Bei der Ausführungsform nach Fig. 3 nimmt der Gewindebolzen 19 den Keil 18 mit, der mit seinen Keilflächen als Bremskeil an der Außenkontur der Scheiben 17, 17' anliegt. Je tiefer der Gewindebolzen 19 eingeschraubt wird, desto höher ist die auf ihn und den Keil 18 wirkende Kraft. Während zwischen der kreisrunden Scheibe 17 und dem Keil 18 eine im wesentlichen gleichbleibende Reibung vorhanden wäre, kommt bei einer Drehung der linken Scheibe 17' entgegen dem Uhrzeigersinn deren sich exzentrisch vergrößernder Außenumfang in eine immer größer werdende Reibung mit dem Keil 18, die gleichzeitig auf die kreisrunde Scheibe 17 übertragen wird, so daß sich zwischen dem Keil und den Scheiben eine stärker werdende Bremskraft bis zur Blockade einstellen würde, würde der Weg der Scheiben nicht durch die unten geschilderten Maßnahmen begrenzt. Durch diese Begrenzung kommt es nach einem Bereich geringer Reibung nach Eingriff eines Teils des exzentrischen Bereichs der linken Scheibe zu einem Bereich größerer Reibung, was sich für den Benutzer so darstellt, daß er bei beginnender Flexion oder Extension zunächst keinen oder nur geringen Widerstand spürt, der vor Erreichen der Endstellung durch ein spürbares Abbremsen abgelöst wird. Die Form des Keils 18 bedingt, daß dieser nur in einer Richtung bremsend wirkt, in der er auf die Scheiben gepreßt wird. In der anderen Richtung, d.h. bei einer Bewegung zurück, übt der Keil keine Bremskraft aus.

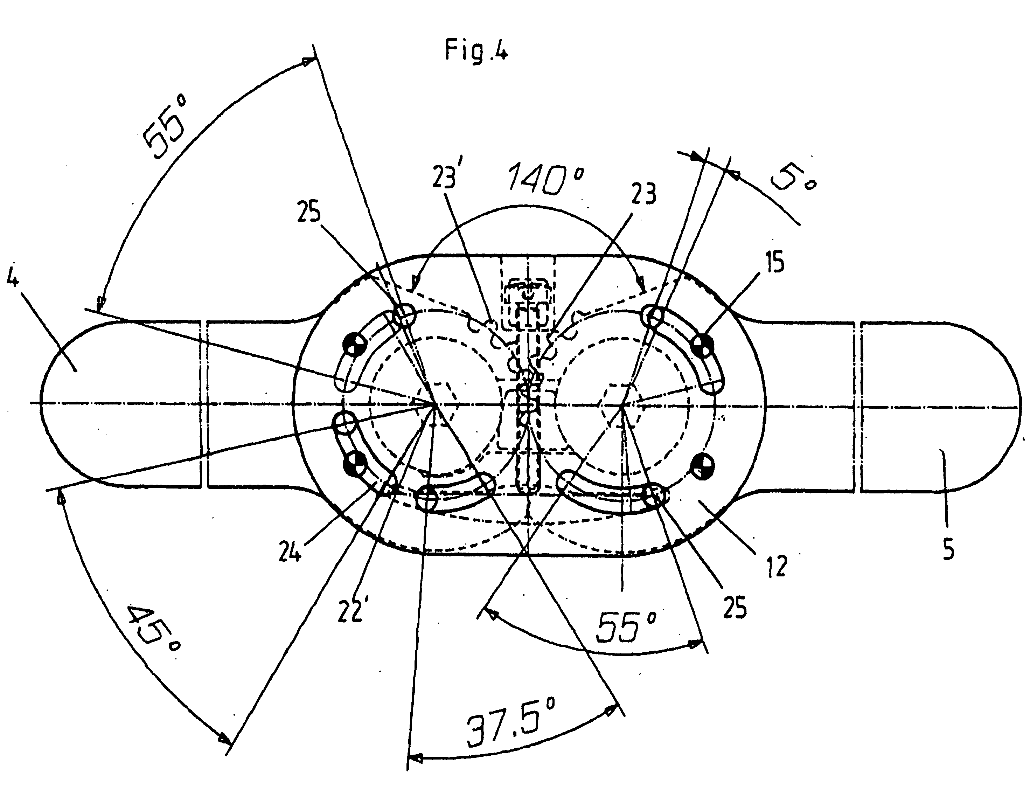

Fig. 4 zeigt die Endstücke der Schienen 4, 5 mit ihren Verzahnungen 23, 23', die miteinander kämmen. Beide Endstücke weisen Sechskantbohrungen 22' auf, mit denen sie, wie geschildert, drehfest mit den (hier nicht gezeigten) Sechskantzapfen verbunden sind. Dadurch sind sie mit der Mitnahme- und Bremsmechanik verbunden, die zu Fig. 3 beschrieben wurde. Auf den Endstücken liegt die dritte Zwischenplatte 12, die eine Anzahl von Bohrungen 25 aufweist, sowie die Enden der unteren Befestigungsstifte 15. Die hier dargestellten Langlöcher 24, die konzentrisch zu den Drehachsen der Enden der Schienen ausgerichtet sind, sind nicht in der Zwischenplatte 12, sondern in den Endstücken der Schienen angeordnet, was nur zur Verdeutlichung zeichnerisch unkorrekt dargestellt ist, um ihr Zusammenwirken mit den Bohrungen 25 zu verdeutlichen. Die Drehachsen der Schienen 4, 5 liegen im wesentlichen in der Längsachse der Sechskantbohrungen 22'. Zur Einstellungen von Endanschlägen für die Schienen 4, 5 können Stifte (Stifte 20 in Fig. 2) durch die Bohrungen 25 gesteckt werden, die in der oberen Abdeckung gehalten werden. Diese Stifte laufen dann in gewünschten Langlöchern, so daß die Wahl von zulässigen Beuge- und Streckwinkeln für das Knie möglich ist. Bestimmte, beispielhafte Winkelbereich sind hier dargestellt. Da die Anordnung von Bohrungen und Langlöchern wie gewünscht ausgeführt werden kann, sind selbstverständlich andere Winkelbereiche möglich. Durch das Zusammenwirken der Mitnahme- und Bremsmechanik mit den Endanschlägen stellt sich der oben genannte Effekt für den Benutzer ein.

## Patentansprüche

1. Orthese mit Orthesengelenk für zwei gegeneinander beug- und streckbare Körperteile, wie Knieorthesen oder Armorthesen,
wobei das Orthesengelenk (7) zwei Schienen (4, 5) drehbar miteinander verbindet, die jeweils mit oberen und unteren Orthesenteilen (2, 3) verbunden sind, die an dem jeweiligen Körperteil befestigbar sind und wobei an der Orthese jeweils lateral und/oder medial ein Schienenpaar mit Orthesengelenk angeordnet ist,
wobei die Schienen endseitig über einen oder mehrere gemeinsame Zapfen oder durch endseitige Verzahnungen (23, 23') im Orthesengelenk (7) miteinander in Eingriff stehen, wobei für Streckung und/oder Beugung einstellbare Endanschläge (20) vorhanden sind,
und wobei die Endanschläge über einen Winkelbereich δ verstellbar sind,
**dadurch gekennzeichnet,**
**daß** die Beugung, bzw. Streckung in dem Orthesengelenk (7) in einem bestimmten Winkelbereich beaufschlagungsfrei erfolgt und in einem anschließenden Winkelbereich α eine Bremsung erfährt, wobei α einen Winkel von zwischen 3° und 25° darstellt,
und **daß** die Bremsung pneumatisch oder hydraulisch erfolgt, wobei ein Mitnehmer (17') ab Erreichen des bestimmten Winkelbereichs ein Pneumatik - oder Hydraulikglied, das an der Orthese gelagert ist, auslöst.

2. Orthese nach Anspruch 1,
**dadurch gekennzeichnet,**
**daß** die Endstücke der Schienen jeweils auf Zapfen einer diese verbindenden Grundplatte drehen.

## Claims

1. An orthosis comprising an orthosis hinge for two body parts which are able to flex and extend in relation to one another, such as knee orthoses or arm orthoses,
wherein the orthosis hinge (7) rotatably joins together two splints (4, 5) joined respectively to upper and lower orthosis parts (2, 3), the said parts being securable on the relevant body part, and wherein a pair of splints with the orthosis hinge is arranged on the orthosis each laterally and/or medially thereof,
wherein the end faces of the splints are in engagement with one another in the orthosis hinge (7) by way of one or more common pivots or by means of teeth (23, 23') on the end face thereof, wherein there are adjustable end stops (20) for extension and/or flexion,
and wherein the end stops are movable over an angular range δ,
**characterised**
**in that** the flexion or extension in the orthosis hinge (7) is effected free of action thereon in a specific angular range, and in a subsequent angular range α experiences braking, α representing an angle of between 3° and 25°,
and **in that** the braking is effected pneumatically or hydraulically, wherein a driver (17') actuates a pneumatic or hydraulic element mounted on the orthosis from when the specific angular range is reached.

2. An orthosis according to Claim 1,
**characterised**
**in that** the end sections of the splints each turn on pivots of a base plate connecting the said splints.

## Revendications

1. Orthèse articulée destinée à deux parties du corps aptes à effectuer un fléchissement et une extension l'une par rapport à l'autre, telle que des orthèses de genou ou des orthèses de bras,
l'articulation (7) de l'orthèse reliant à rotation deux attelles (4, 5) entre elles qui sont reliées respectivement aux parties supérieure et inférieure (2, 3) de l'orthèse qui peuvent être fixées à la partie correspondante du corps et une paire d'attelles articulées étant disposées latéralement et/ou médialement au niveau de l'orthèse, les attelles étant en engagement l'une avec l'autre au niveau des extrémités par un ou plusieurs pivots communs ou par des dentures d'extrémité (23, 23') dans l'articulation (7) de l'orthèse, des butées d'extrémité (20) étant prévues qui sont réglables pour le fléchissement et/ou l'extension, et
les butées d'extrémité étant mobiles dans une plage angulaire δ,
**caractérisée en ce que** le fléchissement respectivement l'extension de l'articulation (7) de l'orthèse sont effectués sans contrainte dans une plage angulaire déterminée et un freinage est subi dans une plage angulaire suivante α, α étant un angle compris entre 3° et 25°,
et **en ce que** le freinage est effectué par des moyens pneumatiques ou hydrauliques, un actionneur (17') déclenchant un élément pneumatique ou hydraulique qui est monté sur l'orthèse lorsque la plage angulaire déterminée a été atteinte.

2. Orthèse selon la revendication 1, **caractérisée en ce que** les pièces d'extrémité des attelles tournent sur des pivots d'une plaque de base reliant ces attelles.
